# EUROPÄISCHE PATENTSCHRIFT

(11) **EP 0 876 328 B1**
(45) Veröffentlichungstag und Bekanntmachung des Hinweises auf die Patenterteilung: **07.06.2000**
(21) Anmeldenummer: 96938197.9
(22) Anmeldetag: 14.11.1996
(51) Int. Cl.: C07C 219/16, C07C 217/08, C07C 233/18, C07C 233/20, C07C 305/10, C07C 309/10, C07C 309/17, C07F 9/09, C11D 1/88, C11D 3/32, C11D 3/33

(54) **AMPHIPHILE VERBINDUNGEN MIT MEHREREN HYDROPHILEN UND HYDROPHOBEN GRUPPEN AUF DER BASIS VON KOHLENSÄUREDERIVATEN**
AMPHIPHILIC COMPOUNDS WITH A PLURALITY OF HYDROPHILIC AND HYDROPHOBIC GROUPS BASED ON CARBONIC ACID DERIVATIVES
COMPOSES AMPHIPHILES A PLUSIEURS GROUPES HYDROPHILES ET HYDROPHOBES A BASE DE DERIVES D'ACIDE CARBONIQUE

(30) Priorität: 20.12.1995 DE 19547643
(43) Veröffentlichungstag der Anmeldung: 11.11.1998
(73) Patentinhaber: RWE-DEA Aktiengesellschaft für Mineraloel und Chemie, 22297 Hamburg (DE)
(72) Erfinder: KOCH, Herbert, D-46282 Dorsten (DE); KWETKAT, Klaus, D-44534 Lünen (DE)
(74) Vertreter: Schupfner, Gerhard D., Dr. Dipl.-Chem.
(86) Internationale Anmeldenummer: EP9604979
(87) Internationale Veröffentlichungsnummer: WO9722577

(56) Entgegenhaltungen:
- EP-A- 0 353 503
- US-A- 5 160 450
- CHEMICAL ABSTRACTS, vol. 101, no. 12, 17.September 1984 Columbus, Ohio, US; abstract no. 92741, TAKEMOTO OIL AND FAT CO., LTD., JAPAN: "Lubricant finishes for synthetic fibers" XP002025375 & JP 59 053 777 A (TAKEMOTO OIL AND FAT CO., LTD., JAPAN)

## Beschreibung

Die Erfindung betrifft amphiphile Verbindungen mit mehreren hydrophilen und hydrophoben Gruppen auf der Basis von Kohlensäurederivaten.

Als amphiphile Substanzen sind eine große Vielfalt an anionischen, kationischen, nichtionischen und zwitterionischen Verbindungen bekannt Die weitaus meisten dieser Substanzen bestehen aus einer hydrophilen Kopfgruppe und wenigstens einem hydrophoben Teil.

Bei den amphiphilen Substanzen gibt es aus ökologischen Gründen, z. B bezüglich der Verringerung des Verpackungs- und Transportaufwandes, die Notwendigkeit, immer größere Wirkung pro Masse an eingesetzter Substanz zu erzielen. Da eine Optimierung durch Mischung von amphiphilen Substanzen nur begrenzt weiterführt, sind neue amphiphile Substanzen mit einem höheren Wirkungsgrad erforderlich. Es müssen daher insbesondere Stoffe mit niedrigeren kritischen Micellbildungskonzentrationen und/oder niedrigeren Grenzflächenspannungen gefunden werden, um die Einsatzmengen an Wirksubstanz deutlich reduzieren zu können. Zudem müssen sie leicht, am besten aus leicht zugänglichen Ausgangssubstanzen, erhältlich sein.

Erste Lösungsansätze in Richtung auf leistungsfähigere amphiphile Substanzen durch Verdoppelung eines Teils der Struktur (hydrophile Kopfgruppe, hydrophobe Gruppe) sind bereits bekannt. So können kationische grenzflächenaktive Verbindungen durch die Addition von langkettigen Alkylhalogeniden an permethylierte Alkylendiamine erhalten werden [R. Zana, M. Benrraou, R. Rueff, Langmuir, 7 (1991) 1072; R. Zana, Y. Talmon, Nature, 362 (1993) 228; E. Alami, G. Beinert, P. Marie, R. Zana, Langmuir, 9 (1993) 1465].

In der EP-A-0 353 503 werden Kohlenfettsäurealkoholester-sulfonate als oberflächenaktive Mittel beschrieben. Sie werden durch Sulfonierung von Kohlenfettsäureestern der Formel

R¹O-(CₘH₂ₘO)ₙ―CO-(OCₘH₂ₘ)ₙ-OR²

(R¹ steht für einen ungesättigten Rest mit 16 - 22 C-Atomen, R² steht für R¹ oder einen gesättigten Rest mit 1 - 22 C-Atomen, m ist 2 und/oder 3, n ist eine Zahl im Bereich von 0 bis 20) sowie anschließende Neutralisation und Hydrolyse der Sulfonierungsprodukte mit Basen hergestellt.

Tenside mit mehreren hydrophilen und hydrophoben Gruppen können aber auf diese Weise nicht erhalten werden.

Weiterhin sind anionische grenzflächenaktive Verbindungen mit wenigstens zwei hydrophilen und wenigstens zwei hydrophoben Gruppen auf der Basis von Diglycidylethern hergestellt worden (US 5 160 450, JP 01 304 033, JP 4 124 165). Diglycidylether gelten jedoch als toxikologisch bedenklich und sind recht teuer. Darüber hinaus wird für ihre Herstellung Epichlorhydrin verwendet, was zu großen Mengen an Reststoffen führt, so daß diese Verbindungen unter ökotoxikologischen wie auch ökonomischen Gesichtspunkten nicht mehr zeitgemäß sind.

Es bestand daher die Aufgabe, amphiphile Verbindungen aufzufinden, die wenigstens zwei hydrophile und wenigstens zwei hydrophobe Gruppen aufweisen, wobei die amphiphilen Verbindungen einen sehr hohen Wirkungsgrad, bezogen auf die Einsatzmenge, haben, und die darüber hinaus aus technisch leicht verfügbaren Rohstoffen ohne unerwünschte Nebenprodukte hergestellt werden können. Die Verbindungen sollten außerdem leicht wieder zu spalten sein.

Die Aufgabe wird erfindungsgemäß durch amphiphile Di-, Oligo- oder Polykohlensäureester, deren Grundkörper aus Di-, Oligo- oder Polykohlensäurederivaten und alkoxylierten Fettaminen bzw. Fettsäureamiden hergestellt werden können, gelöst. Die entsprechenden Di-, Oligo- oder Polykohlensäureester sind nichtionische Tenside, die jedoch weiter zu anionischen amphiphilen Verbindungen umgesetzt werden können. Hier kommen die Sulfierung, Carboxymethylierung und die Umsetzung z. B. zu Isethionaten, Tauraten und Sulfosuccinaten in Frage.

Bei den erfindungsgemäßen amphiphilen Verbindungen handelt es sich daher um Verbindungen der allgemeinen Formel I: 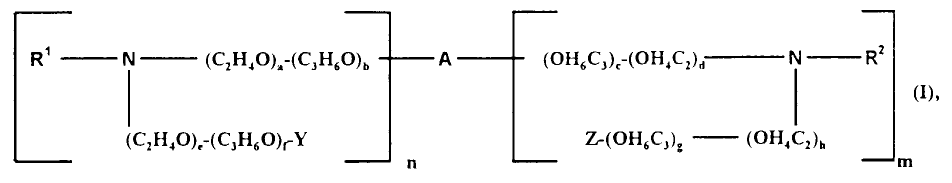 wobei R¹ und R² unabhängig voneinander für einen unverzweigten oder verzweigten, gesättigten oder ungesättigten Kohlenwasserstoffrest mit 1 bis 22, vorzugsweise 6 bis 18, Kohlenstoffatomen oder einen unverzweigten oder verzweigten, gesättigten oder ungesättigten Acylrest mit 2 bis 23, vorzugsweise 7 bis 19, Kohlenstoffatomen stehen, n und m unabhängig voneinander mindestens 1 sind und die Summe aus n und m eine Zahl von 2 bis 200, vorzugsweise 2 bis 100 und besonders bevorzugt 2 bis 10 ist.

a, b, c, d, e, f, g und h bedeuten unabhängig voneinander Zahlen von 0 bis 15, wobei die Summe aus a und b, c und d, e und f sowie g und h jeweils mindestens 1 sein muß und wobei die Alkoxideinheiten statistisch oder blockweise eingebunden sind und die Reihenfolge beliebig ist. Y und Z stehen unabhängig voneinander für Wasserstoff oder für funktionelle Gruppen. Als funktionelle Reste seien -CH₂COOM, -SO₃M, -C₂H₄SO₃M, -C(O)C₂H₃(SO₃M)COOM' und -P(O)(OM)₂,mit M, M' = Alkali, Ammonium-, Alkanolammonium oder ½ Erdalkali, genannt.

Es seien als Substituenten R¹ und R² im einzelnen die Reste Methyl, Ethyl, n-Propyl, n-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, n-Henicosyl, n-Docosyl und ihre verzweigtkettigen Isomeren sowie die entsprechenden einfach, zweifach oder dreifach ungesättigten Reste und die entsprechenden Acylverbindungen genannt

Der Spacer A bedeutet einen aliphatischen, cyclischen oder acyclischen oder einen aromatischen Di-, Oligo- oder Polykohlensäureester der Formel II: 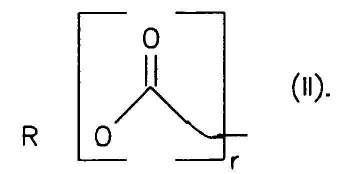 R entstammt einem Di-, Oligo- oder Polyol, das nicht bevorzugt zur Cyclisierung neigt, wobei r Hydroxylgruppen mit Kohlensäurederivaten verestert sind, r steht für eine Zahl von 2 bis 200, bevorzugt von 2 bis 20, besonders bevorzugt für 2 bis 10. Als Beispiele seien hier genannt Alkylendiole, bevorzugt mit 2 bis 8 Kohlenstoffatomen, Di- bis Tetraethylenglykole, Di- bis Tetrapropylenglykole, Polyalkylenglykole wie z. B. Polyethylenglykole, Oligo- oder Polyglycerine, Sorbit, Polyvinylalkohol, Trimethylolpropan und Acetale wie 2,2'-Methylen-bis-(1,3-dioxolan-5-methylen)diol. Aus stöchiometrischen Gründen ist r = n + m.

Die erfindungsgemäßen amphiphilen Verbindungen zeichnen sich durch extrem niedrige kritische Micellbildungskonzentrationen (CMC) und sehr niedrige Grenzflächenspannungen aus, was auf ihre besondere Struktur - wenigstens zwei hydrophile Gruppen und wenigstens zwei hydrophobe Gruppen - zurückgeführt werden muß. Darüber hinaus weisen die meisten von ihnen ein recht hohes hydrophiles Suspendiervermögen auf, das etwa auf halbem Wege zwischen dem konventioneller Tenside und dem des Pentanatriumtripolyphosphats liegt. Einige dieser Verbindungen sind extrem schnelle Netzmittel. Bei allen läßt sich eine sehr hohe Härtestabilität und gute Wasserlöslichkeit feststellen.

Die amphiphilen Verbindungen gemäß dieser Erfindung eignen sich insbesondere als Emulgatoren, Demulgatoren, Detergenzien, Dispergatoren und Hydrotropica sowie Antistatika in Industrie und Haushalt, beispielsweise auf den Gebieten Metallbearbeitung, Erzgewinnung, Textilhilfsmittel, Oberflächenveredelung, Kunststoffverarbeitung, Waschen und Reinigen, Reinigen harter Oberflächen, dabei insbesondere als manuelles Geschirrspülmittel, Kosmetik, Medizin und Nahrungsmittelverarbeitung und -zubereitung.

Hierbei können sie mit allen gängigen anionischen, nichtionischen, kationischen und ampholytischen grenzflächenaktiven Substanzen kombiniert werden.

Als Beispiele für nichtionische grenzflächenaktive Substanzen, die für eine Kombination eingesetzt werden können, seien Fettsäureglyceride, Fettsäurepolyglyceride, Fettsäureester, Ethoxylate höherer Alkohole, Polyoxyethylenfettsäureglyceride, Polyoxyethylenpropylenglykolfettsäureester, Polyoxyethylensorbitanfettsäureester, Polyoxyethylen-Rhizinusöl- oder gehärtete Rhizinusöl-Derivate, Polyoxyethylenlanolinderivate, Polyoxyethylenfettsäureamide, Polyoxyethylenalkylamine, Alkanolamine, Alkyl-aminoxide, Derivate von Eiweißhydrolysaten, Hydroxymischether, Alkylpolyglycoside, Aminoxide und Alkylglucamide genannt.

Als Beispiele für anionische grenzflächenaktive Substanzen, die für Kombinationen eingesetzt werden können, seien Seifen, Ethercarbonsäuren und deren Salze, Alkylsulfonate, α-Olefinsulfonate, a-Sulfofettsäurederivate, Sulfonate höherer Fettsäureester, höhere Alkoholsulfate, Alkoholethersulfate, Hydroxymischethersulfate, Salze von Phosphatestern, Tauride, Isethionate, lineare Alkylbenzolsulfonate, Cumolsulfonat, Alkylarylsulfonate, Sulfate der Polyoxyethylenfettsäureamide und Salze von Acylaminosäuren genannt.

Als Beispiele für gängige kationische grenzflächenaktive Substanzen, die für Kombinationen eingesetzt werden können, seien Alkyltrimethylammoniumsalze, Dialkyldimethylammoniumsalze, Alkyldimethylbenzylammoniumsalze, Alkylpyridiniumsalze, Alkylisochinoliniumsalze, Benzethoniumchloride und kationische Acylaminosäurederivate genannt.

Als Beispiele für ampholytische grenzflächenaktive Substanzen, die für Kombinationen eingesetzt werden können, seien Aminosäuren, Betaine, Sulfobetaine, Imidazolinderivate, Sojaöllipide und Lecithin genannt.

Darüber hinaus können die erfindungsgemäßen amphiphilen Verbindungen auch für sich miteinander kombiniert werden.

Den erfindungsgemäßen amphiphilen Verbindungen können ebenfalls gängige Additive zugesetzt werden. Solche Additive werden speziell für eine Formulierung ausgewählt und umfassen beispielsweise anorganische Salze, wie Natriumchlorid und -sulfat, sowie Builder, Hydrotropica, UV-Absorber, Weichmacher, Chelatbildner, Viskositätsmodifizierer und Riechstoffe.

Die obengenannten Verbindungen lassen sich aus Di-, Oligo- oder Polykohlensäureestern und mindestens zwei Äquivalenten alkoxylierter Fettamine und/oder alkoxylierter Fettsäureamide herstellen. Als Vorstufe dienen hier die Di-, Oligo- oder Polyole, die pro Hydroxygruppe mit einem Äquivalent Kohlensäurediethylester umgesetzt werden können. Es müssen wenigstens zwei Äquivalente Kohlensäurediethylester sein, bei Oligo- oder Polyolen lassen sich die Moleküleigenschaften durch den Umsetzungsgrad mit Kohlensäureester, respektive Amin- oder Amidethoxylat im letzten Schritt, genauer einstellen. Für beide oben beschriebenen Veresterungs- bzw. Umesterungsreaktionen können bekannte Katalysatoren , wie z. B. Titanate, Gemische von Antimontrioxid und Calciumacetat, Stannane, Zinkacetat und Alkalialkoholate, eingesetzt werden. Titanate haben sich jedoch als besonders günstig hinsichtlich der Reaktionszeiten und Farbqualität erwiesen. Die anionischen Amphiphile lassen sich dadurch herstellen, daß man die obengenannten Produkte z. B. mit Amidosulfonsäure, Chloressigsäuresalzen, Isethionaten oder Maleinsäureanhydrid umsetzt und mit wäßrigen Alkali- oder Erdalkalihydroxiden, wäßrigem Ammoniak oder Alkanolaminen neutralisiert. Bei Bedarf werden die Produkte in wäßriger Lösung mit Wasserstoffperoxid (0,1 bis 2,0 %, bezogen auf Feststoff) gebleicht.

## Patentansprüche

1. Amphiphile Verbindungen der allgemeinen Formel I 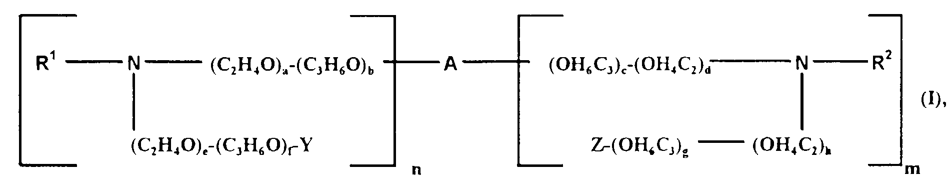 wobei R¹ und R² unabhängig voneinander einen unverzweigten oder verzweigten, gesättigten oder ungesättigten Kohlenwasserstoffrest mit 1 bis 22 Kohlenstoffatomen oder einen unverzweigten oder verzweigten, gesättigten oder ungesättigten Acylrest mit 2 bis 23 Kohlenstoffatomen, A einen Di-, Oligo- oder Polykohlensäureester und Y und Z Wasserstoff oder einen funktionellen Rest -CH₂COOM, -SO₃M, -C₂H₄SO₃M, -C(O)-C₂H₃(SO₃M)COOM' oder -P(O)(OM)₂ mit M, M' = Alkali, Ammonium-, Alkanolammonium oder ½ Erdalkali bedeuten und wobei n und m unabhängig voneinander jeweils mindestens 1, die Summe aus n und m 2 bis 200 und a, b, c, d, e, f, g und h unabhängig voneinander 0 bis 15, wobei die Summe aus a und b, c und d, e und f sowie g und h jeweils mindestens 1 sein muß, sind.

2. Amphiphile Verbindungen nach Anspruch 1,
dadurch gekennzeichnet,
daß der Spacer A der allgemeinen Formel (II) 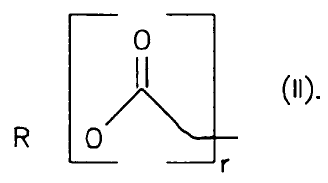 genügt, R aus einem Di-, Oligo- oder Polyol, bevorzugt aus Di-, Tri- und Polyethylenglykolen stammt, und r für eine Zahl von 2 bis 200 steht.

3. Amphiphile Verbindungen nach Anspruch 1 oder 2,
dadurch gekennzeichnet,
daß die Kohlenwasserstoffreste R¹ und R² in der Formel I unabhängig voneinander 6 bis 18 Kohlenstoffatome und die Acylreste unabhängig voneinander 7 bis 19 Kohlenstoffatome enthalten.

4. Amphiphile Verbindungen nach einem der vorhergehenden Ansprüche als Gemisch von Homologen und/oder als Gemisch von Homologen verschiedener amphiphiler Verbindungen.

5. Verwendung der amphiphilen Verbindungen nach einem der Ansprüche 1 bis 4 als Emulgatoren oder Demulgatoren.

6. Verwendung der amphiphilen Verbindungen nach einem der Ansprüche 1 bis 4 als Verarbeitungshilfsmittel bei der Metallbearbeitung, Erzgewinnung, Oberflächenveredelung, Kunststoffherstellung oder Kunststoffverarbeitung.

7. Verwendung der amphiphilen Verbindung nach einem der Ansprüche 1 bis 4 als Textilhilfsmittel oder für das Reinigen und Waschen von Textilien.

8. Verwendung der amphiphilen Verbindungen nach einem der Ansprüche 1 bis 4 für das Reinigen von harten Oberflächen in Haushalt und Gewerbe, inbesondere für das manuelle Reinigen von Geschirr.

9. Verwendung der amphiphilen Verbindungen nach einem der Ansprüche 1 bis 4 für das Reinigen und Waschen von Haut und Haar.

## Claims

1. Amphiphilic compounds of the general formula I 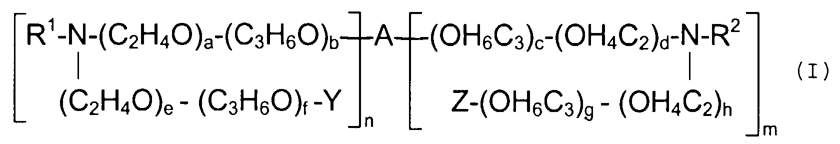 where **R**^{**1**} and **R**^{**2**}, independently of one another, are an unbranched or branched, saturated or unsaturated hydrocarbon group having 1 to 22 carbon atoms, or an unbranched or branched, saturated or unsaturated acyl group having 2 to 23 carbon atoms, **A** is a di-, oligo-, or polycarbonate, and **Y** and **Z** are hydrogen or a functional group -CH₂COOM, -SO₃M, -C₂H₄SO₃M, -C(O)-C₂H₃(SO₃M)COOM', or -P(O)(OM)₂, wherein **M**, **M'** are equal to an alkali, ammonium, alkanol ammonium, or % alkaline earth ion, and where each **n** and **m**, independently of one another, is at least 1, the total of n and m is from 2 to 200, and **a**, **b**, **c**, **d**, **e**, **f**, **g**, and **h**, independently of one another, are from 0 to 15, wherein the total of a and b, c and d, e and f, and g and h must be at least 1 each.

2. Amphiphilic compounds according to claim 1,
**characterized in that**
the spacer **A** corresponds to the general formula (II) 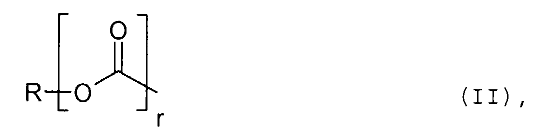 **R** is derived from a di-, oligo-, or polyol, preferably from di-, tri-, and polyethylene glycols, and **r** represents a number from 2 to 200.

3. Amphiphilic compounds according to claim 1 or 2,
**characterized in that**
the hydrocarbon groups **R**^{**1**} and **R**^{**2**} in formula I contain independently of one another 6 to 18 carbon atoms, and the acyl groups contain independently of one another 7 to 19 carbon atoms.

4. Amphiphilic compounds according to any one of the preceding claims as a mixture of homologues and/or a mixture of homologues of different amphiphilic compounds.

5. Use of the amphiphilic compounds according to any one of claims 1 to 4 as emulsifiers or demulsifiers.

6. Use of the amphiphilic compounds according to any one of claims 1 to 4 as auxiliaries in metal working, ore mining, surface finishing, plastics production, or plastics processing.

7. Use of the amphiphilic compounds according to any one of claims 1 to 4 as textile auxiliaries or for cleaning and washing textiles.

8. Use of the amphiphilic compounds according to any one of claims 1 to 4 for cleaning hard surfaces in households or trade, particularly for the manual washing of dishes.

9. Use of the amphiphilic compounds according to any one of claims 1 to 4 for cleaning and washing skin and hair.

## Revendications

1. Composés amphiphiles de formule générale I : 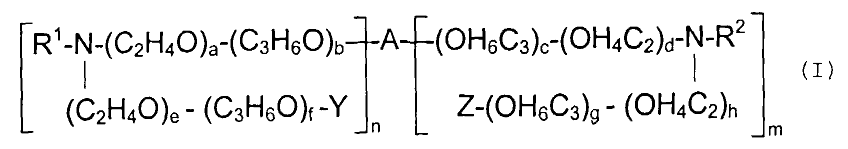 dans laquelle R¹ et R² représentent, indépendamment l'un de l'autre, un radical hydrocarboné non ramifié ou ramifié, saturé ou insaturé, ayant 1 à 22 atomes de carbone ou un radical acyle non ramifié ou ramifié, saturé ou insaturé, ayant 2 à 23 atomes de carbone, A un ester de diacide, d'oligoacide ou de polyacide carbonique, et Y et Z de l'hydrogène ou un radical fonctionnel -CH₂COOM, -SO₃M, -C₂H₄SO₃M, -C(O)-C₂H₃(SO₃M)COOM¹ ou -P(O)(OM)₃ avec M, M¹ = métal alcalin, ammonium, alcanolammonium ou ½ métal alcalino-terreux, et n et m doivent être, indépendamment l'un de l'autre, au moins de 1, la somme de n et m de 2 à 200 et a, b, c, d, e, f, g et h indépendamment les uns des autres de 0 à 15, la somme de a et b, c et d, e et f ainsi que g et h, indépendamment les uns des autres, respectivement d'au moins 1.

2. Composés amphiphiles selon la revendication 1, caractérisés en ce que l'élément d'espacement A répond à la formule générale (II) : 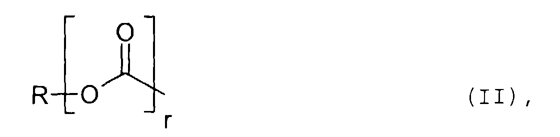 R est tiré d'un di-, oligo- ou polyol, de préférence de di-, tri- et polyéthylèneglycols, et r est un nombre de 2 à 200.

3. Composés amphiphiles selon la revendication 1 ou 2, caractérisés en ce que les radicaux hydrocarbonés R¹ et R² de la formule I contiennent, indépendamment l'un de l'autre, 6 à 18 atomes de carbone et les radicaux acyle, indépendamment l'un de l'autre, 7 à 19 atomes de carbone.

4. Composés amphiphiles selon l'une quelconque des revendications précédentes sous la forme d'un mélange d'homologues et/ou d'un mélange d'homologues de divers composés amphiphiles.

5. Utilisation des composés amphiphiles selon l'une quelconque des revendications 1 à 4 comme émulsionnants ou désémulsionnants.

6. Utilisation des composés amphiphiles selon l'une quelconque des revendications 1 à 4 comme auxiliaires de traitement dans le traitement des métaux, l'extraction des minerais, l'ennoblissement de surfaces, la fabrication de matières plastiques ou la transformation de matières plastiques.

7. Utilisation des composés amphiphiles selon l'une quelconque des revendications 1 à 4 comme auxiliaire textile ou pour le nettoyage et le lavage de textiles.

8. Utilisation des composés amphiphiles selon l'une quelconque des revendications 1 à 4 pour le nettoyage de surfaces dures dans les domaines domestiques et industriels, en particulier pour le nettoyage manuel de la vaisselle.

9. Utilisation des composés amphiphiles selon l'une quelconque des revendications 1 à 4 pour le nettoyage et le lavage de la peau et des cheveux.
